# EUROPEAN PATENT APPLICATION

(11) **EP 4 389 307 A1**
(43) Date of publication of application: **26.06.2024**
(21) Application number: 22214485.9
(22) Date of filing: 19.12.2022
(51) Int. Cl.: B06B 1/02, G01H 11/06, G01N 29/26

(54) **RING ARRAY TRANSDUCER**

(71) Applicant: Koninklijke Philips N.V., 5656 AG Eindhoven (NL)
(72) Inventor: DIRKSEN, Peter, Eindhoven (NL); SHULEPOV, Sergei Y., Eindhoven (NL); TIMMERMANS, Petrus Henricus Maria, 5656AG Eindhoven (NL); VAN SCHAIJK, Rob, Eindhoven (NL); DE WIJS, Willem-Jan Arend, Eindhoven (NL)
(74) Representative: Philips Intellectual Property & Standards

(57) **Abstract**

A transducer ring array for volumetric ultrasound imaging. The ring array comprises a set of first channels, each first channel comprising an outer CMUT drum and an inner CMUT drum connected via a radiofrequency, RF, line. A biasing system, for each first channel, is used to apply a biasing voltage to either the outer CMUT drum or the inner CMUT drum of the first channels. The ring array also comprises a set of second channels, each second channel comprises an outer CMUT drum. The outer CMUT drums of both the first channels and the second channels form an outer ring with an outer diameter, and the inner CMUT drums of the first channels form an inner ring with a smaller, inner, diameter.

## Description

### FIELD OF THE INVENTION

The invention relates to the field of ultrasound transducers. In particular, the invention relates to the field of ring array transducers.

### BACKGROUND OF THE INVENTION

Ring array transducers enable volumetric imaging while using a relatively low number of channels compared to conventional two-dimensional, 2D, array transducers. This means that fewer cables have to be connected to the transducer. More generally, a ring array is thought to be the simplest transducer geometry that combines three-dimensional, 3D, imaging with a low number of channels. Thus, it proves possible to get a 3D image using, for example, only 64 channels as the computational load is reduced with the lower number of elements.

For example, in Choe et. al. "GPU-based real-time volumetric ultrasound image reconstruction for a ring array", IEEE Transactions on Medical Imaging, 18 Mar 2013, 32(7):1258-1264, DOI: 10.1109/tmi.2013.2253117, a ring array is shown with 64 elements capable of 3D imaging. The ring array shown has a radius of 1.16mm with an imaging depth of 2mm - 15mm.

However, such an imaging depth is typically not enough for external imaging/monitoring applications. Further, it has been realized that the focal region of such a ring is limited and may not meet the requirements for external applications, such as fetal monitoring.

### SUMMARY OF THE INVENTION

The invention is defined by the claims.

According to examples in accordance with an aspect of the invention, there is provided a transducer ring array for volumetric ultrasound imaging, the ring array comprising:
a set of first channels, wherein each first channel comprises an outer CMUT drum and an inner CMUT drum connected via a radiofrequency, RF, line;
a biasing system for each first channel for applying a biasing voltage to either the outer CMUT drum or the inner CMUT drum; and
a set of second channels, wherein each second channel comprises an outer CMUT drum,
wherein the outer CMUT drums of both the first channels and the second channels form an outer ring with a same outer diameter, and the inner CMUT drums of the first channels form an inner ring with a smaller, inner, diameter.

Ring arrays enable 3D ultrasound imaging with a low number of channels. However, the focal region of ring is limited and often does not meet the requirements for certain applications (e.g., fetal monitoring).

As such, it is proposed to generate multi-ring arrays while still having a low number of channels. For double-ring arrays, this can be achieved by having two different types of channels in the array. The first channels have at least one outer CMUT drum and at least one inner CMUT drum. The second channels have only an outer CMUT drum.

The outer CMUT drums of both the first and second channels form an outer ring (e.g., similar to a single-ring array) while the inner CMUT drums of the first channel form a second, inner, ring. A biasing system is used to activate either the outer CMUT drums (for faraway imaging) or the inner CMUT drums (for nearby imaging). As such, the focal region of the ring array is increased without the need to add additional channels.

The first channels can be used to transmit/receive ultrasound signals from either the outer CMUT drums or the inner CMUT drums.

The CMUT drums may be placed on a substrate (e.g., a printed circuit board, PCB). The substrate may be a planar rigid substrate or a flexible substrate. When placed on a flexible substrate, the ring array may be arranged/placed on a curved structure.

The first channels may comprise two or more outer CMUT drums and the outer CMUT drums further from the inner CMUT drum may have a larger diameter than the CMUT drums closer to the inner CMUT drum.

Having the diameter of CMUT drums increase towards the outer edges of the ring array improves the packing density of the ring array. This is possible because the center frequency of a CMUT can be approximated as being independent of the diameter provided the other parameters (e.g., bias voltage) are constant.

The first channels may comprise two or more inner CMUT drums and the inner CMUT drums closer to the outer CMUT drums may have a larger diameter than the CMUT drums further from the outer CMUT drums.

The first channels may comprise three or more outer CMUT drums and three or more inner CMUT drums and the second channels may comprise three of more outer CMUT drums.

Having three or more CMUT drums per channel in both the inner and outer rings increases the transmit and receive signal strength, thereby leading to an increase in the quality of the ultrasound data obtained.

The biasing system may comprise two individually addressable connections for each first channel, one for the outer CMUT drum and one for the inner CMUT drum.

The biasing system may comprises a bias switching system on each first channel for switching between applying a biasing voltage to the outer CMUT drum and the inner CMUT drum of the first channels.

The invention also provides an ultrasound imaging system comprising:
the transducer ring array; and
a controller configured to:
   obtain nearby ultrasound data from the inner CMUT drums of the first channels by applying a bias to the inner CMUT drums;
   obtain faraway ultrasound data from the outer CMUT drums of the first channels and the outer CMUT drums of the second channels by applying a bias to the outer CMUT drums of the first channels; and
   generate a compound, three dimensional, ultrasound image by combining the nearby ultrasound data and the faraway ultrasound data.

Obtaining faraway ultrasound data may also comprise applying a bias to the outer CMUT drums of the second channels.

The invention also provides a method of manufacturing a transducer ring array, the method comprising:
generating ring array portions on a silicon wafer, the ring array portions comprising at least one first channel, each first channel comprising an outer CMUT drum and an inner CMUT drum, and at least one second channel, each second channel comprising an outer CMUT drum; and
combining two or more ring array portions to generate the transducer ring array.

It has been realized that manufacturing ring shapes results in poor yields and high usage of silicon. As such, it is proposed to manufacture portions of the ring array and then combine the portions. This means that less silicon is used overall, and any deficient portions can be discarded individually, instead of having to discard the whole ring array.

The method may further comprise placing the ring array portions on a substrate in a ring pattern, the substrate comprising an RF line for each first channel, for connecting the outer CMUT drum and the inner CMUT drum of the corresponding first channel, and a biasing system for each of the first channels for applying a biasing voltage to either the outer CMUT drum or the inner CMUT drum of each first channel.

The substrate may also comprise an RF line for each second channel, connected to the outer CMUT drum of corresponding second channel, and a biasing system for each of the second channels for applying a biasing voltage to the outer CMUT drum of the corresponding second channel.

The method may further comprise combining four or more ring array portions to generate the transducer ring array.

The method may further comprise separating the ring array portions from the silicon wafer by using deep reactive ion etching, DRIE, to etch the silicon wafers and grinding the etched silicon wafers to release the ring array portions.

This is particularly advantageous when the ring array portions are non-rectangular (e.g., when the ring array portions are curved).

Of course, if the ring array portions are, for example, rectangular, the ring array portions can be obtained from the silicon wafer via dicing.

The method may further comprise testing the ring array channels to identify acceptable ring array portions and combining two or more of the acceptable ring portions to generate the transducer ring array.

Combining the two or more ring array portions may comprise placing the ring array portions in a ring pattern.

Combining the two or more ring array portions may comprise placing the ring array portions on a substrate, the substrate comprising an RF line connecting the outer CMUT drum and the inner CMUT drum of each first channel and a biasing system for each of the first channels for applying a biasing voltage to either the outer CMUT drum or the inner CMUT drum of each first channel.

The substrate may be a flexible or non-flexible (e.g., flat) printed circuit board (PCB), depending on the end application requirements. For example, the substrate may be flexible such that it can be wrapped around a curved shape.

The biasing system may comprise two individually addressable connections for each first channel, one for the outer CMUT drum and one for the inner CMUT drum.

The biasing system may comprise a bias switching system on each first channel for switching between applying a biasing voltage to the outer CMUT drum and the inner CMUT drum of the first channels.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a sketch of a single ring array;
Fig. 2 shows contours of the focal regions for single ring arrays with different diameters;
Fig. 3 illustrates the concept of a multi ring array;
Fig. 4 shows a double ring array with 64 elements;
Fig. 5 shows part of a ring array with a dense pack ring array geometry;
Fig. 6 shows a silicon wafer with ring elements; and
Fig. 7 shows an assembled ring array with four ring array portions.

### DETAILED DESCRIPTION OF EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a transducer ring array for volumetric ultrasound imaging. The ring array comprises a set of first channels, each first channel comprising an outer CMUT drum and an inner CMUT drum connected via a radiofrequency, RF, line. A biasing system, for each first channel, is used to apply a biasing voltage to either the outer CMUT drum or the inner CMUT drum of the first channels. The ring array also comprises a set of second channels, each second channel comprises an outer CMUT drum. The outer CMUT drums of both the first channels and the second channels form an outer ring with an outer diameter, and the inner CMUT drums of the first channels form an inner ring with a smaller, inner, diameter.

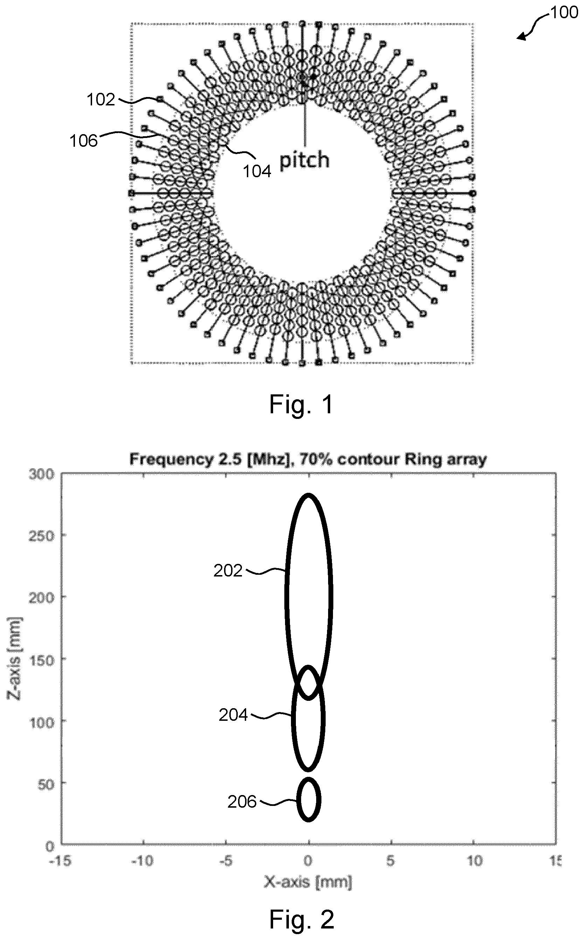

Fig. 1 shows a sketch of a single ring array 100. The single ring array 100 has 64 channels 102, where each channel 102 has five CMUT drums 10 connected with a radio frequency, RF, line 106. As such, only the 64 channels have to be analyzed.

There is an interest in monitoring solutions for pregnancies. In particular, there is an interest in monitoring solutions for high-risk pregnancies with preexisting complications.

However, the single ring array 100 has been found to have a limited focal region, thereby making it unsuitable for fetal imaging/monitoring. As a rule of thumb, the average pitch between the CMUT drums 104 limits the maximal center frequency for a ring array. It has been found that for a single ring array with a focal region up to around 280mm, a ring diameter of around 12mm is needed. Keeping the number of channels 102 to 64 means that the maximum center frequency is limited to around 2.2. MHz (assuming a two-pulse excitation and 40% bandwidth - acceptable for monitoring purposes).

The pitch generally refers to the period of a periodic structure. In this case, the distance between adjacent CMUT drums is called the pitch. For a ring array, the pitch (distance) is not a constant: outer CMUT elements are at a larger distance compared to the inner. However, assuming a `thin' ring array, one may use the average pitch.

The relation between pitch, operating frequency and image quality is well known, both from an experimental point of view and theoretically. For a phased array, the general rule is that the pitch should be smaller than the wavelength *λ* in order to avoid grating lobes that spoil the image quality.

Typically the pitch is chosen as smaller than half the wavelength *λ*. However, usually a somewhat larger pitch is still allowed. For example a pitch of 0.7*λ* is also used.

In this case, the wavelength corresponds to an upper-corner wavelength *λᵤₚ*. One often excites a transducer in a pulsed mode, the frequency spectrum having a certain width result. The central frequency is called *F_{c}*, the upper-corner frequency is called *Fᵤₚ* and the lower corner frequecny is called *F_{low}*. The bandwidth corresponds to the frequency difference between *Fᵤₚ* and *F_{low}*. A high bandwidth means that the transducer responds to a large range in frequency, which provide a good and crisp image.

Frequency and wavelength are related: *F* = *λc*, where c is the speed of sound. As such, a high frequency means a small wavelength, which also means a smaller pitch is needed. For water, the speed of sound is approximately 1500 meter/sec. Thus, the higher the desired upper corner frequency is, the smaller the pitch is required to be.

However, because of the limited focal region, such a single ring array would be limited to a minimum depth of around 120mm. For fetal monitoring, a penetration depth between ∼280mm and ~80mm is required.

Fig. 2 shows contours 202, 204 and 206 of the focal regions for single ring arrays with different diameters. The x-axis shows a lateral distance relative to the single ring array (in mm). The z-axis shows a depth relative to the single ring array (in mm).

The contours are line of equal amplitude (70% of the maximum) of the absolute value of the pressure. The 70% contour indicated is generally of interest as this part is most suitable for imaging. Inside this contour, the pressure is at maximum.

The contour 202 shows the focal region for a single ring array with a diameter of 28mm at a frequency of 2.5Mhz. The contour 204 shows the focal region for a single ring array with a diameter of 20mm at a frequency of 2.5Mhz. The contour 206 shows the focal region for a single ring array with a diameter of 12mm at a frequency of 2.5Mhz.

As can be seen, none of the contours shown provided a large enough focal region for fetal monitoring/imaging (i.e., covering ~80mm to ~280mm).

The focal regions for single ring arrays with diameters of 26mm, 24mm, 16mm and 14mm were also determined (not shown). None of the single ring arrays proved sufficient for fetal monitoring (i.e., having a focal region covering ~80mm to ~280mm).

As such, there is a need for an improved solution which provides a higher focal region (e.g., for fetal monitoring) whilst keeping the number of channels to a minimum.

It is proposed to provide a multi-ring array where the CMUT drums of the inner ring(s) are connected to the same RF line as the part of the CMUT drums of the outer ring such than some channels comprise CMUT drums of the inner ring(s) and CMUT drums of the outer ring. A biasing system is then provided to either bias the CMUT drums of the outer ring or the CMUT drums of the inner ring. The outer ring and the inner ring(s) will have different diameters such that the multi-ring array essentially has two (or more) focal regions. As such, the combination of the focal regions provides an increased overall focal region.

This results in an effective focal region comprised of a combination of the contours shown in Fig. 2. For example, the contour 202 (having a diameter of 28mm) and the contour 204 (having a diameter of 20mm), when combined, could cover a focal region between ~80mm to ~280mm.

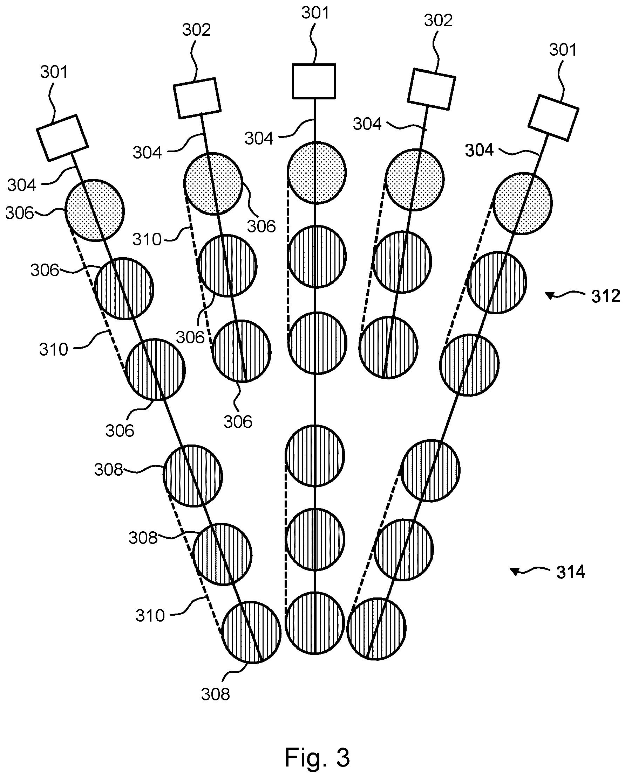

Fig. 3 illustrates the concept of a multi ring array. In particular, Fig. 3 shows part of a double ring array. Five channels are shown, three first channels 301 and two second channels 302. In this example, the first channels 301 have an RF line 304 connected to six CMUT drums; three outer CMUT drums 306 and three inner CMUT drums 308. The outer CMUT drums 306 are all connected to a biasing line 310 for applying a bias to the outer CMUT drums 306. The inner CMUT drums 308 are also connected to a biasing line 310 for biasing the inner CMUT drums 308. As such, a bias can be applied to either the outer CMUT drums 306 or the inner CMUT drums 308.

The second channels 302 have three outer CMUT drums 306, all connected to a biasing line 310 for applying a bias to the outer CMUT drums 306. The outer ring 312 is formed of the outer CMUT drums 306 of both the first channels 301 and the second channels 302 and the inner ring 314 is formed of the inner CMUT drums 308 of the first channels 301.

The outer ring 312, having an outer diameter, will have a first focal region, and the inner ring 314, having a smaller inner diameter, will have a second focal region. As such, ultrasound data obtained from both of the rings can be combined to provide ultrasound data for a larger, combined, focal region.

Any of the CMUT drums can be used in the collapsed or non-collapsed mode.

Bias switching between the rings can be used to select the appropriate ring. Next the data may can be combined into a single compound image. Alternatively, an independent bias line can be used for the outer CMUT drums 306 and the inner CMUT drums 308. It is noted that bias switching may need fewer connections than having independent bias lines.

A biasing system using bias switching involves a circuit capable of only providing a bias to either the outer CMUT drums 306 or the inner CMUT drums 308 of the first channels 301.

A double or multiple ring array can thus provide nearby imaging (with the inner ring 314) and far away imaging (with the outer ring 312). For nearby imaging, the bias is turned on for the inner CMUT drums 308 and turned off for the outer CMUT drums 306. In other words, during nearby imaging, it is as if only the CMUT drums of the inner ring 314 are active and the CMUT drums of the outer ring 312 are not present at all.

For far away imaging, the bias is turned off for the inner CMUT drums 308 and is only turned on for the outer CMUT drums 306. Compound ultrasound data can thus be obtained by combining the ultrasound data obtained from the nearby imaging and the far away imaging. For example, a compound 3D image can be generated by combining the images from nearby imaging the far away imaging. The compound ultrasound data/image will thus be able to over the focal regions of both the outer ring 312 and the inner ring 314.

Thus, a double or multiple ring array solves the focal region issue. Additionally, the diameters of the outer ring 312 and the inner ring 314 can be selected to cover, for example, an entire penetration depth of 50mm to 250mm without increasing the complexity of the electronics (i.e., the number of channels remains low).

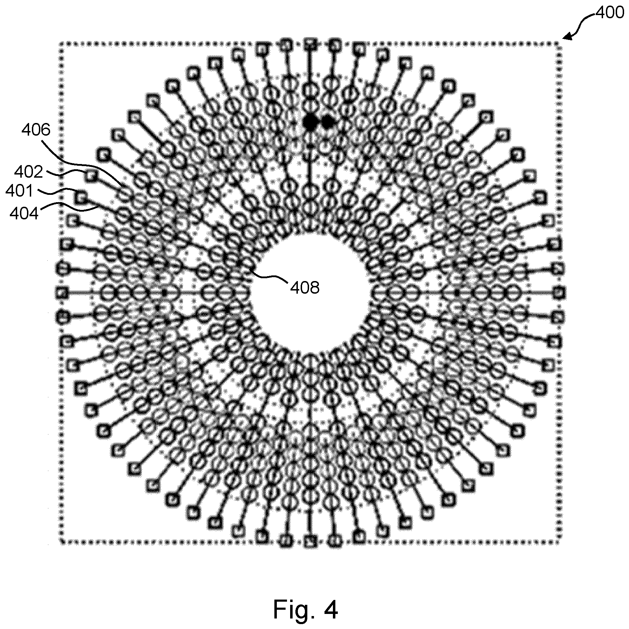

Fig. 4 shows a double ring array 400 with 64 elements. The double ring array 400 has 32 first channels 401 and 32 second channels 402. The first channels 401 each have five outer CMUT drums 406 and three inner CMUT drums 408 connected via an RF line. The second channels 402 each have five outer CMUT drums 406 connected via an RF line 404. A biasing system (not shown) is used which can bias either the outer CMUT drums 406 or the inner CMUT drums 408 of the first channels 401. As such, the double ring array 400 can be switched between using the outer ring (i.e., the outer CMUT drums 406 of both the first channels 401 and the second channels 402) or the inner ring (i.e., the inner CMUT drums 308 of the first channels 401).

The double ring array 400 includes the integration of scanning CMUT MEMS ultrasound transducer. Because a single US transducer plane is not able to cover the whole womb, multiple US transducers (e.g., distributed over the abdomen for fetal monitoring) are used and are integrated in a conformal patch and/or smart textile. The double ring array can thus be used to identify fetus movement, position, anatomy and, detects mother and fetus heart rate due to its larger overall focal range.

As previously discussed, a single ring array will not cover the required focal depth for fetal monitoring. This issue is solved by using a double ring, where either the inner ring is activated by switching on the bias voltage or the outer ring is activated. Thus, a larger depth of focus is obtained. Note that the number of channels is not increased and is fully determined by the outer ring.

Note that, for Fig. 4, an open circle represents a single CMUT drum (e.g., having a typical diameter of about 350 microns in the case of low frequency CMUT drums ~ 2.5MHz). The squares on each channel represent bond pads. Not all components are shown (e.g., the bias lines).

A multi-ring array can be used in both professional and non-professional settings (e.g., for a remote monitoring at home).

Note that Fig. 4 shows a flat double ring array. However, the ring array may be placed on a flexible substrate such that it can be wrapped around a curved structure.

A `dense pack ring array' geometry is further proposed to improve the performance.

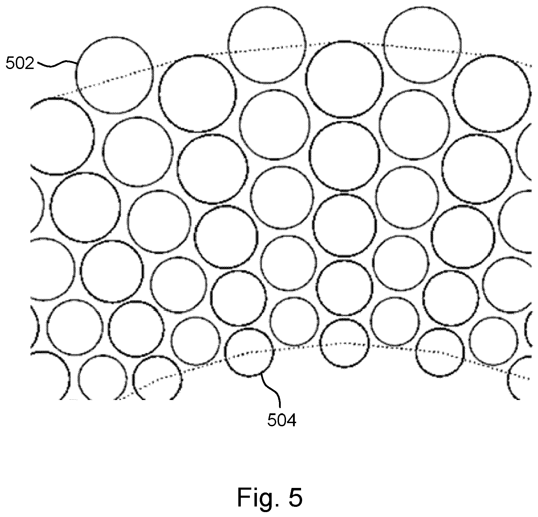

Fig. 5 shows part of a ring array with a dense pack ring array geometry. The packing density of a ring array may be improved by having a CMUT diameter that increases towards the rim of the array. In other words, the CMUT drums 502 further from the center of the ring(s) have a larger diameter than the CMUT drums 504 closer to the center of the ring.

To a fair approximation, the center frequency of a CMUT is independent of the diameter, provided all other parameters including the bias voltage are a constant. This is true for a standard collapse mode operated CMUT drum as well as an annular ring CMUT operated in collapse mode.

Additionally, the shape of the channels may be altered to improve the image quality. For example, it is known that using a spiral shape can reduce the number of artifacts in the resulting data, thereby providing improved image quality. More generally, the shape of the channels may be curved, the diameter of the CMUTs may be variable and/or the length of the channels may be variable.

See, for example, Li, X., Gachagan, A., & Murray, P. (2020). Design of 2D sparse array transducers for anomaly detection in medical phantoms. Sensors 2020, 20(18), [5370]. https://doi.org/10.3390/s20185370 where various array configurations/shapes are discussed.

Each channel preferably has more than 3 CMUT drums as this provides a higher signal strength per channel for both transmit and receive.

The manufacturability of the large outer rings, having a diameter up to 30mm could result in loss of yield. A manufacturing method is therefore proposed to remedy the situation, where ring array portions are preferentially etched out of the wafer (DRIE) or separated by means of for example a dicing saw or stealth dicing. Tested and selected ring array portions can thus be assembled by means of a pick and place technique onto a substrate.

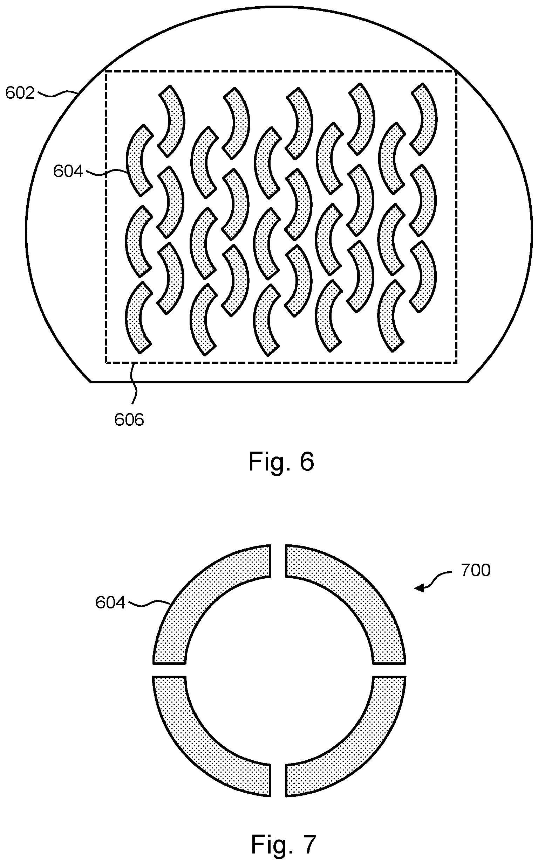

Fig. 6 shows a silicon wafer 602 with ring elements 604. Ring array portions 604 are fabricated in a dense pack manner on the silicon wafer 602. The area in between the segments may be filled by `dummy CMUT structures' to improve the processing uniformity. For example, the area 604 may be filled with either dummy CMUT structures or with the CMUT drums in the areas comprising the ring array portions 604. Dummy CMUT structures may not have connections to other CMUT structures or may not have electrodes.

For example, DRIE etching is used to separate the segments to enable curved ring array portions 604 to be obtained. DRIE etching can be combined with back side grinding to separate the ring array portions on the silicon wafer. After testing, the ring array portions are combined on a substrate by a `pick and place' system, to assemble the ring array.

Fig. 7 shows an assembled ring array 700 with four ring array portions 604. The substrate is not shown. More than four ring arrays may be preferred in order to improve the packing of the ring array portions 604 on the silicon wafer.

Drawbacks of manufacturing ring arrays typically include low yields and high usage of silicon.

The proposed assembly overcomes the typical drawbacks of manufacturing ring array because the ring array elements 604 are individually picked and placed in the ring pattern. This increases the yield whilst reducing the use of silicon.

The arrangement may, for example, use square wafer fragments. A complete circular window can be placed over the ring array portions 604 in order to form a homogeneous face to the part to be imaged.

The substrate (e.g., a PCB) may be made of a non-flexible or a flexible substrate, depending on the end application requirements.

It will be appreciated that this manufacturing method can be used for both single ring arrays and multi-ring arrays (e.g., double ring arrays).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

The term CMUT used in this application stands for capacitive micromachined ultrasonic transducer.

If the term "adapted to" is used in the claims or description, it is noted the term "adapted to" is intended to be equivalent to the term "configured to".

Any reference signs in the claims should not be construed as limiting the scope.

## Claims

1. A transducer ring array for volumetric ultrasound imaging, the ring array comprising:
a set of first channels (301), wherein each first channel comprises an outer CMUT drum (306) and an inner CMUT drum (308) connected via a radiofrequency, RF, line (304);
a biasing system (310) for each first channel for applying a biasing voltage to either the outer CMUT drum or the inner CMUT drum; and
a set of second channels (302), wherein each second channel comprises an outer CMUT drum,
wherein the outer CMUT drums of both the first channels and the second channels form an outer ring (312) with a same outer diameter, and the inner CMUT drums of the first channels form an inner ring (314) with a smaller, inner, diameter.

2. The transducer ring array of claim 1, wherein the first channels comprise two or more outer CMUT drums and wherein the outer CMUT drums further from the inner CMUT drum have a larger diameter than the CMUT drums closer to the inner CMUT drum.

3. The transducer ring array of claim 2, wherein the first channels comprise two or more inner CMUT drums and wherein the inner CMUT drums closer to the outer CMUT drums have a larger diameter than the CMUT drums further from the outer CMUT drums.

4. The transducer ring array of any of claims 1 to 3, wherein the first channels comprise three or more outer CMUT drums and three or more inner CMUT drums and wherein the second channels comprise three of more outer CMUT drums.

5. The transducer ring array of any of claims 1 to 4, wherein the biasing system comprises two individually addressable connections for each first channel, one for the outer CMUT drum and one for the inner CMUT drum.

6. The transducer ring array of any of claims 1 to 4, wherein the biasing system comprises a bias switching system on each first channel for switching between applying a biasing voltage to the outer CMUT drum and the inner CMUT drum of the first channels.

7. An ultrasound imaging system comprising:
the transducer ring array of any of claims 1 to 6; and
a controller configured to:
obtain nearby ultrasound data from the inner CMUT drums of the first channels by applying a bias to the inner CMUT drums;
obtain faraway ultrasound data from the outer CMUT drums of the first channels and the outer CMUT drums of the second channels by applying a bias to the outer CMUT drums of the first channels; and
generate a compound, three dimensional, ultrasound image by combining the nearby ultrasound data and the faraway ultrasound data.

8. A method of manufacturing a transducer ring array, the method comprising:
generating ring array portions (604) on a silicon wafer (602), the ring array portions comprising at least one first channel (301), each first channel comprising an outer CMUT drum (306) and an inner CMUT drum (308), and at least one second channel (302), each second channel comprising an outer CMUT drum; and
combining two or more ring array portions to generate the transducer ring array.

9. The method of claim 8, further comprising combining four or more ring array portions to generate the transducer ring array.

10. The method of claims 8 or 9, further comprising separating the ring array portions from the silicon wafer by using deep reactive ion etching, DRIE, to etch the silicon wafers and grinding the etched silicon wafers to release the ring array portions.

11. The method of any of claims 8 to 10, further comprising testing the ring array channels to identify acceptable ring array portions and combining two or more of the acceptable ring portions to generate the transducer ring array.

12. The method of any of claims 8 to 11, wherein combining the two or more ring array portions comprises placing the ring array portions in a ring pattern.

13. The method of any of claims 8 to 12, wherein combining the two or more ring array portions comprising placing the ring array portions on a substrate, the substrate comprising an RF line (304) connecting the outer CMUT drum and the inner CMUT drum of each first channel and a biasing system (310) for each of the first channels for applying a biasing voltage to either the outer CMUT drum or the inner CMUT drum of each first channel.

14. The method of claim 13, wherein the biasing system comprises two individually addressable connections for each first channel, one for the outer CMUT drum and one for the inner CMUT drum.

15. The method of claim 13, wherein the biasing system comprises a bias switching system on each first channel for switching between applying a biasing voltage to the outer CMUT drum and the inner CMUT drum of the first channels.
